(19) Europäisches Patentamt
European Patent Office
Office européen des brevets

(11) EP 4 223 882 A1

(12) EUROPEAN PATENT APPLICATION

(43) Date of publication:
09.08.2023 Bulletin 2023/32

(21) Application number: 22154819.1

(22) Date of filing: 02.02.2022

(51) International Patent Classification (IPC):
C12P 7/58 (2006.01)        C12N 9/04 (2006.01)

(52) Cooperative Patent Classification (CPC):
C12P 7/58; C12N 9/0006

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Vilnius University
01513 Vilnius (LT)

(72) Inventors:
• Laurynenas, Audrius
  Vilnius (LT)
• Tetianec, Lidija
  Vilnius (LT)
• Bratkovskaja, Irina
  Vilnius (LT)
• Casaite, Vida
  Vilnius (LT)
• Stankeviciute, Jonita
  Vilnius (LT)
• Meskys, Rolandas
  Vilnius (LT)
• Dagys, Marius
  Vilnius (LT)

(74) Representative: Petniunaite, Jurga
AAA Law
P.O. Box 33
A. Gostauto street 40B
03163 Vilnius (LT)

(54) PROCESS FOR PRODUCING ALDONIC ACIDS

(57) The present invention relates to enzymatic process for producing aldonic acids of mono-, di- and oligosaccharides and their mixtures using PQQ-dependent dehydrogenase, and mediator thionine, and mediator reoxidation system in the reactor equipped with continuous supplement of hydrogen peroxide, substrate and alkali. The use of the dehydrogenase to oxidize substrates in bioreactors is very attractive due to its broad substrate specificity and high catalytic activity. The reoxidation of the dehydrogenase performed by effective mediator dependent enzymatic method. Mediator thionine is highly reactive with the dehydrogenase, both forms of the mediator - oxidized and reduced are stable, nontoxic and cheap.

Fig. 1.

EP 4 223 882 A1

## Description

### Technical field

This invention is related to the field of bienzymatic systems and production of aldonic acids.

### Background art

There are not many types of biomolecules, which sources are as inexhaustible as carbohydrates are. This abundance is a direct consequence of vastness of the structural diversity and biological functions. Naturally, carbohydrates have found numerous applications starting with food and feed stock and ending with clothing. Further uses and new applications for carbohydrates, however, heavily depend on the existing technology for transformation of the available compounds into more valuable derivatives.

One class of such derivatives are aldonic acids, which are produced by oxidising respective aldoses. For example, glucose and lactose oxidation products - gluconic and lactobionic acids - have found numerous applications in pharmaceutical, food and cosmetics industries due to antioxidant, antimicrobial, moisturizing and chelating properties as well as prebiotic effects. However, the further growth of present and future applications is constrained by existing methods of aldonic acids synthesis.

At present, aldonic acids are produced via several main methods, which are very unequal in a production capacity and quality of the products. The chemical and electrochemical oxidations allow a production of large amounts of aldonic acids but usually using harmful and expensive metal catalysts, which leave heavy metal traces in products and the presence of nonspecific oxidation products. These reactions typically proceed in strong alkali medium and need the expense of energy (electrochemical oxidation) (US2007/0027341, US5132452, US4843173, US7982031).

Fermentation with microorganisms is much more environmentally friendly and it allows production of substantial amounts of the wanted compound at the expense of much slower rates of production and more complex purification steps (US3862005, JP2001245657, JP5100987, WO2010/106230, US9315834). In addition, microorganisms such as *Acinetobacter* and *Burkholderia* are not sufficiently safe, and there are problems in using aldonic acid produced using these microorganisms for food and feed applications. The enzymatic oxidation method is possibly the most desirable from the viewpoint of production efficiency, purity of products and environmental concerns (JP2007535331, JP2005504554, JP6321857).

The enzymatic method for production of aldonic acids heavily relies on the availability of a biocatalyst capable to oxidise aldoses. Some of them, for example glucose and lactose oxidases, are quite specific enzymes, which preferably oxidise their namesake sugars (US 2020/0239511). The by-product of the oxidation reaction is hydrogen peroxide, which is readily converted back to dioxygen with catalase (JP2005504554, JP6321857). However, the substrate specificity of oxidases and available catalysis rates limit production of aldonic acids. Therefore, there is an initiative to find more versatile enzymes, which can oxidise a wide range of sugars.

Patent EP1753297 describes using carbohydrate oxidase and maintaining pH at a stable value during the carbohydrate oxidase based enzymatic conversion of lactose into lactobionic acid, which produces higher yield and/or a reduced reaction time in the enzymatic conversion. The conversion of lactose to lactobionic acid without pH control was 41 %, while with pH control the conversion was 100 %. This process uses carbohydrate oxidase and is adapted only for lactose containing dairy substrate (e.g. milk, whey).

Isolated enzymes are quite expensive and for their use in large-scale industrial production the effective methods of applications must be invented. Review [Gluconic Acid Production, Recent Patents on Biotechnology 2007, Vol. 1, No. 2] summarises the methods of gluconic acid production. The enzymatic methods presented in the review suffer from the low effectivity of enzyme application - 1206 g of sodium gluconate can be obtained with 1 g of soluble glucose oxidase. The better yield was obtained with immobilized glucose oxidase-catalase composition (US4460686), but the specificity of the glucose oxidase limits the application of the method only for glucose conversion to gluconic acid.

It is worth to mention, that concentration of oxygen in the reactor can limit the rate of the process catalysed by oxidases. In the case of whey lactose enzymatic conversion to lactobionic acid the concentration of oxygen in the reactor mixture decreases from 5.82 mg/L at start to 0.50 mg/L after 5 min (EP1753297).

Thus, the presented enzymatic methods for aldonic acid production are limited for specific oxidase and its substrate - carbohydrate and the oxygen supply to the reactor.

### Summary of the Invention

Here we present a new scheme for synthesis of aldonic acids based on PQQ-dependent dehydrogenase. The used enzyme has wide substrate specificity and high catalysis rate. The enzyme is dehydrogenase and reacts very slowly with oxygen. The mediators can be used instead to regenerate the oxidised form of the enzyme. In our scheme a stable, efficient and recoverable mediator was used in the enzyme reoxidation scheme coupled with heme peroxidase or catalase. The terminal electron acceptor in such scheme may be oxygen, but the most efficient was hydrogen peroxide. To avoid the inactivation of the enzymes with high concentrations of hydrogen peroxide the dozing of the compound was controlled. No enzyme

- mediator system for bioproduction of aldonic acids with glucose dehydrogenase has been described so far. The reaction of synthesis occurs at mild conditions - water solution and ambient temperature. Various saccharides can be oxidised - mono-, di- and oligosaccharides
- to the corresponded aldonic acids by applying the invented method. The method allows even the oxidation of the mixture of the saccharides. The method is very effective - the degree of conversion of saccharides and the turnover number of the dehydrogenase were very high, and the turnover number of PQQ-dependent dehydrogenase in the model reactor reaches $\sim 1 \times 10^7$. This turnover number corresponds the production of -23 kg of potassium gluconate using 1 g of the enzyme.

The process of producing of aldonic acids performed in a custom sensor-controlled bioreactor with PQQ-dependent dehydrogenase, mediator and mediator reoxidation system. The reoxidation of the dehydrogenase performed by effective mediator thionine. Thionine is highly reactive with the dehydrogenase, both forms of the mediator - oxidized and reduced - are stable, nontoxic and cheap. The oxidized mediator form produced in reaction with dioxygen or hydrogen peroxide and heme peroxidase (horseradish peroxidase). Heme peroxidase exhibits high catalytic activity at pH 7.0 and broad substrate specificity. It uses hydrogen peroxide as co-substrate to oxidase mediators. In presented method the hydrogen peroxide can be used as terminal electron acceptor. We have developed the method to supply the hydrogen peroxide to the reaction mixture to ensure the high process rate and avoid the hydrogen peroxide-induced enzyme inactivation. The addition of hydrogen peroxide to the reactor mixture was performed in very small doses (1 $\mu$mol) by using the precise dispenser developed by our team. The rate of dosage was controlled by analysing the data of our custom highly sensitive hydrogen peroxide and optical, oxidized mediator form, sensors. The concentration of the substrate in the invented process was optimized to ensure the high reaction rate. The present invention allows high yield production of aldonic acids which are useful material in food, pharmaceuticals and industrial fields.

**Brief description of figures**

Fig. 1 shows enzymatic mediator dependent schemes of substrate oxidation with the enzymes of the method embodiments. A - scheme with hydrogen peroxide as terminal electron acceptor, B - scheme with dioxygen as terminal electron acceptor.

Fig. 2. shows extracted ion chromatograms of oxidation products of (A) cellobiose, $[M-H]^-=357$; (B) deoxyribose, $[M-H]^-=149$; (C) ribose, $[M-H]^-=165$; (D) xylose, $[M-H]^-=165$; (E) glucose, $[M-H]^-=195$; (F) lyxose, $[M-H]^-=165$; (G) lactose, $[M-H]^-=357$; (H) galactose, $[M-H]^-=195$.

Fig. 3. shows the dependences of the PQQGDH catalysed reaction rate on substrate concentration (cellobiose - A, xylose - B). Points are experimentally determined rate of reaction and the line is the approximation of data with the equation of enzymatic reaction with inhibition of enzyme with substrate rate dependence on substrate concentration.

**Detailed description of the Invention**

The present invention is an enzymatic method for the synthesis of the aldonic acids of mono-, di-, oligosaccharides and their mixtures. In the method we used the combination of PQQ-dependent dehydrogenase, mediator and mediator oxidation system. The oxidation process was performed in a custom sensor-controlled reactor.

For oxidation of the carbohydrates in the present invention PQQ-dependent dehydrogenase was used. In a preferred embodiment, the PQQ-dependent dehydrogenase is obtained from the source disclosed by Olsthoorn and Duine [1996, Arch. Biochem. Biophys., 336(1), 42]. This enzyme - glucose dehydrogenase (PQQGDH) has broad substrate specificity and high catalytic activity. The use of the enzyme to oxidize carbohydrates in bioreactors is very attractive, but the application requires effective enzyme reoxidation method. In this work we invoked the mediator dependent dehydrogenase reoxidation method. The mediator in regeneration scheme must be highly reactive with the enzyme to regenerate, both forms of the mediator - oxidized and reduced must be stable, nontoxic, and cheap. In the invention we present the mediator thionine (3,7-Diamino-5λ4-phenothiazin-5-ylium) for use in combination with dehydrogenase. The reduced form of the thionine is leuco thionine. This form can be oxidized in reaction with dissolved oxygen or enzymatically. The rate of oxidation of the mediator by oxygen is low, but the oxidation catalysed by enzymes proceeds at much higher rates. In the preferred embodiment of the method, we used heme peroxidase to catalyse the oxidation of reduced form of mediator.

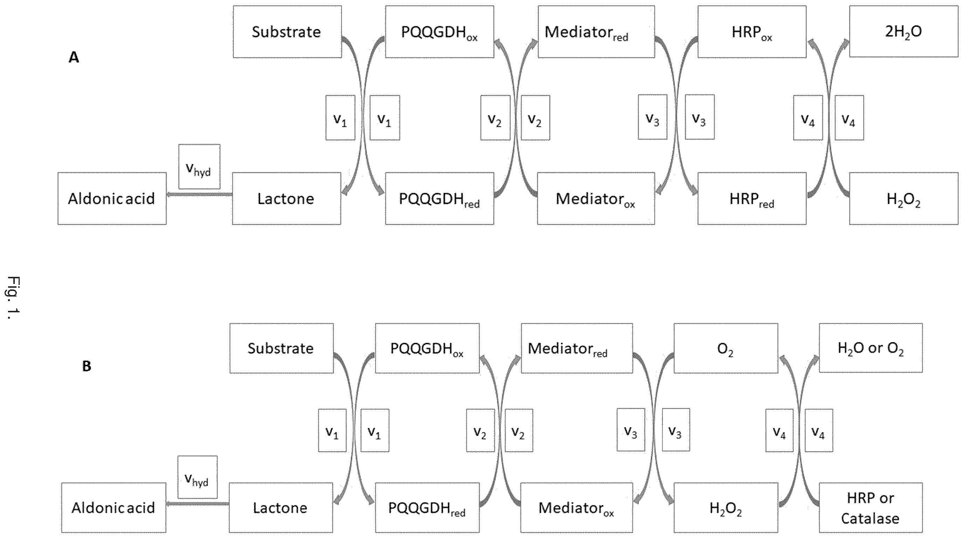

Heme peroxidases show high catalytic activity and broad substrate specificity. The peroxidases use hydrogen peroxide as co-substrate to oxidase mediators. In the most preferred embodiment, we used peroxidase from horseradish. For peroxidase from horseradish (HRP) the comprehensive data on the reactivity of the enzyme with hydrogen peroxide and various substrates are available. The bimolecular horseradish peroxidase and leuco thionine reactivity constant is $4.8 \times 10^8$ $M^{-1}s^{-1}$. The schemes of the conversions of carbohydrates with terminal electron acceptor hydrogen peroxide or dioxygen and with the enzymes of the preferred embodiments are presented in Fig. 1.

In a presented embodiment of the enzymatic process, we used enzymes in soluble form, but the immobilized enzymes can also be used for the invented process.

In a preferred embodiment we used hydrogen peroxide as terminal electron acceptor. Hydrogen peroxide can negatively act on the activity of enzymes - in the absence of electron donor each molecule of HRP catalyses the dismutation of 1380 $H_2O_2$ molecules till it is inactivated. To enable the application of heme peroxidase for reoxidation of the mediator the addition of hydrogen peroxide to the reactor mixture must be controlled. In this work we applied the homemade precise dispenser which controlled by the hydrogen peroxide sensor with the sensitivity in the range from 0 to 100 $\mu$M and with optical thionine sensor. During the progress of reaction in reactor the current of the amperometric hydrogen peroxide sensor and the current of optical sensor were registered. The optical sensor current corresponds to the concentration of thionine, and the current of hydrogen peroxide sensor corresponds to hydrogen peroxide concentration in reactor. The rate of dosage of hydrogen peroxide was regulated to maintain the concentration of oxidized mediator at 95-100 % level of total mediator and to not to allow the concentration of hydrogen peroxide to exceed the 20 $\mu$M level.

As is common in biotechnical reactions, the pH-value of the reaction liquid must be kept in a range favourable for the enzymes. In a preferred embodiment the pH of the reactor mixture was kept at pH 7 or pH 8. Potassium hydroxide for neutralization of reaction product - aldonic acid was added via automatic dispenser. The rate of potassium hydroxide solution dosage was regulated considering the measurement of pH with pH electrode.

The concentration of the substrate in the invented process must be optimized to ensure the high reaction rate. The concentration was optimized considering the dependence of the enzyme and substrate reaction rate on substrate concentration. The dependence of the rate allows to take into account the inhibition of the enzyme by the excess of the substrate and to calculate the concentration of the substrate at which the reaction of enzymatic conversion proceeds at the maximal velocity. According to this dependence the addition of substrate to the reactor performed in two different ways. Substrate at high concentration (in the range from 80 mM to 220 mM) was added at start for batch type reactors, while the continuous supplement of the substrate via automatic dispenser was used in the reactors of fed-batch type. The substrate in fed-batch reactors was dozed at the same rate as hydrogen peroxide to compensate the substrate consumption equimolar to hydrogen peroxide addition.

In a further embodiment of the invented method glucose oxidation was performed without adding of $H_2O_2$. Leuco thionine can be reoxidized by oxygen at slow rate, so the dioxygen was the terminal electron acceptor in the reactors. For decomposition of hydrogen peroxide formed in the reaction of leuco dye and dioxygen, either peroxidase, or catalase was used. The pH of the reaction mixture was kept at the desired value by adding the potassium hydroxide via automatic dispenser. The rate of potassium hydroxide solution dosage was regulated considering the measurement of pH with pH electrode. The enzyme turnover number was calculated assuming the amount of product in the reactor is equal to the amount of potassium hydroxide added. The substrate was supplied to the fed-batch reactor at the same rate as potassium hydroxide.

The following examples are provided only for the purpose of illustrating the invention and are not intended to be limiting in any manner.

### *PQQ-dependent glucose dehydrogenase preparation and activity measurement*

Recombinant *Acinetobacter calcoaceticus* PQQ-dependent GDH (PQQ-sGDH) was purified as described in [A. J. Olsthoorn, J. A. Duine, Arch. Biochem. Biophys., 336(1), 42 (1996)]. The enzyme solution was prepared using deionized water and the molar concentration was calculated assuming the molecular mass of the enzyme to be 100 kDa.

The activity of PQQ-sGDH was measured by standard DCPIP-PMS assay. The reaction mixture contained 60 $\mu$M of phenazine methosulfate (PMS), 40 $\mu$M of 2,6-dichloroindophenol sodium salt hydrate (DCPIP), 20 mM glucose, 12 nM of *Aspergillus niger* catalase (ANC) and the appropriate amount of the enzyme in 50 mM phosphate buffer solution, pH 7.0. In this mixture glucose was enzyme reducing substrate, PMS - primary electron acceptor, DCPIP - terminal electron acceptor. The absorbance of DCPIP was registered at 600 nm of wavelength. The concentration of DCPIP was calculated using molar extinction coefficient 19.6 $mM^{-1}cm^{-1}$. One unit of PQQGDH activity corresponds to the amount of the enzyme which catalyse the reduction of 1 $\mu$mol of DCPIP per 1 minute. The specific activity of the PQQGDH used in this work was 1900 U/mg.

*Heme peroxidase and catalase activity measurements*

The activity of Horseradish (HRP) peroxidase was measured by observing the rate of the oxidation of DCPIP. The reaction mixture contained 100 $\mu$M of $H_2O_2$, 60 $\mu$M DCPIP and the appropriate amount of the enzyme. The absorbance of DCPIP was registered at 600 nm of wavelength. The concentration of DCPIP was calculated using molar extinction coefficient 19.6 mM$^{-1}$cm$^{-1}$. One unit of HRP activity corresponds to the amount of the enzyme which catalyse the oxidation of 1$\mu$mol of DCPIP per 1 minute. The specific activity of the horseradish peroxidase used in this work was 103 U/mg.

The activity of the *Aspergillus niger* catalase (ANC) enzyme was measured in 50 mM phosphate buffer solution, pH 7.0 by observing spectrophotometrically at 240 nm of wavelength the decomposition of hydrogen peroxide (initial concentration 12 mM). The activity of the enzyme suspension was 3500 U/ml.

*Detection of saccharide oxidation products*

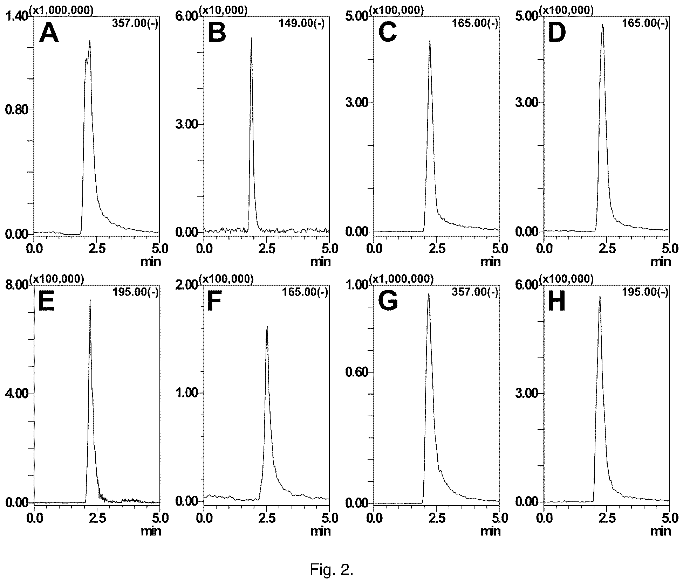

After the conversion of saccharides in the model reactor, the samples were collected and analysed using high-performance liquid chromatography-mass spectrometry (HPLC-MS) system equipped with a mass spectrometer with an electrospray ionization (ESI) source. The chromatographic separation was conducted using a YMC Pack Pro column (3 × 150 mm; YMC) at 40°C and a mobile phase that consisted of 0.1% formic acid water solution (solvent A) and acetonitrile (solvent B) delivered in the isocratic (60% solvent B) elution mode. Mass scans were measured from m/z 50 up to m/z 1200 at a 350°C interface temperature, 250°C desolvation line (DL) temperature, ±4500 V interface voltage, and neutral DL/Qarray, using $N_2$ as nebulizing and drying gas. Mass spectrometry data were acquired in both positive and negative ionization modes. Based on HPLC-MS analysis, the conversion of mono- and disaccharides resulted in the formation of a single product with a molecular mass that was 16 Da higher than that of the substrate (Fig. 2). In the reaction mixtures sampled before enzymatic oxidation, we did not identify any molecular ions typical for oxidation products.

*The reactivity of thionine with PQQGDH*

The reaction of the thionine and reduced PQQGDH was investigated spectrophotometrically by registering the decrease of absorbance of thionine at wavelength of 600 nm (for thionine concentrations from 2.6 $\mu$M to 15 $\mu$M) or of 500 nm (for thionine concentrations from 36 $\mu$M to 120 $\mu$M). Glucose (10 mM) was used as the enzyme reducing substrate. The dependence of the thionine reduction rate on its initial concentration was analysed and the bimolecular rate constant was calculated as described in [Tetianec et al., Appl. Biochem. Biotechnol., 163, 404 (2011)]. The bimolecular thionine and the reduced PQQGDH reactivity constant estimated to be equal to $(2.8\pm0.4)\times10^6$ M$^{-1}$s$^{-1}$. The value of the thionine constant is similar to the values of medium reactive electron acceptors, such as DCPIP, and lower than the value of high reactive electron acceptors - PMS, cation radicals of organic compounds. Analysis of reaction rate at high thionine concentrations and 10 mM of glucose revealed that there is no enzyme inhibition by thionine at its concentrations up to 0.41 mM.

*The parameters of reactivity of saccharide substrates and PQQGDH and the optimization of the saccharide concentration in the conversion reactor.*

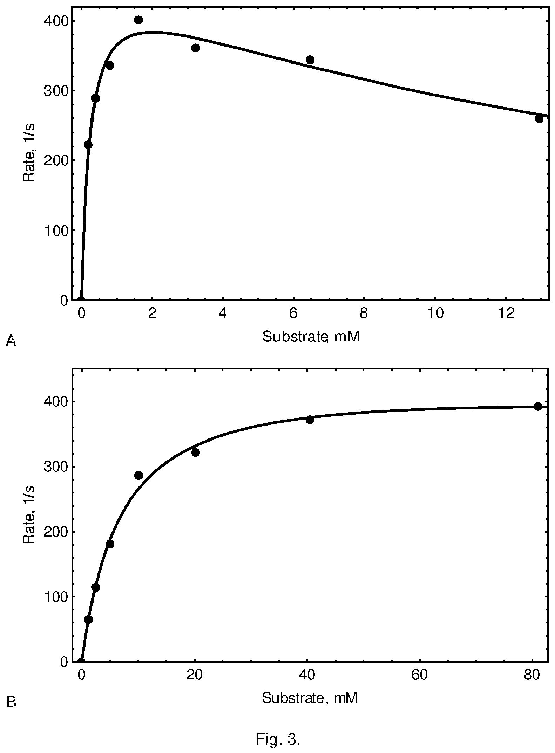

The dependence of PQQGDH catalysed substrate oxidation rate of substrate concentration was estimated for various substrates at thionine concentration of 220 $\mu$M (Fig. 3). The dependence of thionine reduction rate on carbohydrate concentration was analysed by applying the equation X1 and the apparent parameters $K_M$, $K_i$ and $V_{max}$ were calculated:

$$v = \frac{s \cdot V_{max}}{K_M + s \cdot (1 + \frac{s}{K_i})} \qquad (X1)$$

The ratio of $V_{max}$ with Km was considered as the parameter specifying the reactivity of the various substrates - the higher the value of the ratio - the better substrate reactivity. Ki - is the enzyme inhibition by the substrate constant.

The concentration of the substrate at which the rate of thionine reduction was maximal ($_{Cmax}$) was calculated as $\sqrt{K_M \cdot K_i}$. The calculated apparent parameters of various substrates oxidation presented in Table 1. It is worth to notice that for glucose and other substrates the parameters at higher thionine concentration (400 $\mu$M) increased too (Tables 1 and 2). Thus, the increase of the mediator concentration allows to increase the oxidation reaction rate and to diminish the inhibition of the enzyme by substrate.

The investigated substrates were divided into 2 groups according to the substrate concentration at which the maximal reaction rate was observed ($c_{max}$): 1) $c_{max}<20$ mM (glucose, lactose, galactose, cellobiose); 2) $c_{max}>20$ mM (lyxose, xylose, ribose, deoxyribose). The substrates of the first group showed higher reactivity with PQQGDH and lower values of $K_i$ than those parameters for substrates of second group. For the substrates of 1 group to increase the rate of conversion in reactor its concentration should be maintained at $c_{max}$ by adding the substrate as it consumed in the oxidation reaction. This type of the reactor is a fed-batch reactor. For low reactive substrates of second group to ensure the effective conversion rate large amount of substrate (concentration in the range from 80 mM to 220 mM) should be added at the start of the reactor. This type of the reactor is a batch reactor.

Table 1. Apparent parameters of saccharide substrate oxidation with PQQGDH at pH 7.0 and fixed thionine concentration (220 $\mu$M or 410 $\mu$M marked with asterisk).

| Substrate | $V_{max}$, 1/s | $K_M$, mM | $K_i$, mM | $V_{max}/K_M$, $mM^{-1}S^{-1}$ | $c_{max}$, mM |
|---|---|---|---|---|---|
| Deoxyribose | 28±5 | 72±12 | >$10^4$ | 0.39±0.13 | >$10^4$ |
| Ribose | 250±60 | 21±5 | 330±80 | 12±5 | 84±39 |
| Xylose | 460±20 | 7.1±0.3 | 1090±50 | 64±6 | 88±8 |
| Lyxose | 190±40 | 220±40 | >$10^4$ | 0.87±0.35 | >$10^4$ |
| Galactose | 350±30 | 2.4±0.2 | 150±10 | 150±20 | 19±3 |
| Glucose | 400±40 | 0.67±0.07 | 24±2 | 600±120 | 4.0±0.8 |
| Lactose | 380±50 | 1.5±0.1 | 25±2 | 260±50 | 5.2±0.9 |
| Cellobiose | 320±20 | 0.50±0.02 | 11±1 | 640±60 | 2.4±0.2 |
| Glucose* | 520±30 | 0.56±0.03 | 42±2 | 920±110 | 4.9±0.6 |
| Galactose* | 349±2 | 1.8±0.1 | 870±10 | 200±3 | 39±1 |
| Lactose* | 430±10 | 0.61±0.02 | 51±2 | 700±50 | 6.4±0.4 |
| Cellobiose* | 470±10 | 0.50±0.02 | 21±1 | 930±50 | 3.3±0.2 |

Table 2. Apparent parameters of glucose oxidation with PQQGDH at pH 7.0 and thionine concentration of 410 $\mu$M.

| Buffer solution pH | $CaCl_2$, mM | $V_{max}$, 1/S | $K_M$, mM | $K_i$, mM | $V_{max}/K_M$, $mM^{-1}s^{-1}$ | $c_{max}$, mM |
|---|---|---|---|---|---|---|
| 7.0 | 5.0 | 520±30 | 0.56±0.03 | 42±2 | 920±110 | 4.9±0.6 |
| 8.0 | 5.0 | 440±30 | 0.53±0.04 | 66±5 | 840±130 | 5.9±0.9 |
| 7.0 | 100 | 610±40 | 0.43±0.03 | 110±10 | 1400±200 | 7.6±1.0 |
| 8.0 | 100 | 700±30 | 0.56±0.02 | 56±2 | 1300±100 | 5.6±0.4 |

Example 1

Glucose conversion reactors with hydrogen peroxide as terminal electron acceptor

The conversion of glucose performed in the model fed-batch reactor. The specific conditions for each run of the reactor are listed in Table 3. The amount of the horseradish peroxidase was 1.6 mg (165 U) for each run. At the reactor start the period of hydrogen peroxide dosage was from 1 to 3 seconds depending on the conditions used. During the reaction progress the period increased (usually up to 1.5 times). The amount of product formed during the reaction time assumed equal to the amount of added hydrogen peroxide. This amount was used to calculate the turnover number of the PQQGDH and evaluate the efficiency of the process (Table 3). The higher yield of the product and higher turnover numbers of the enzyme were achieved if higher mediator concentration was used at pH 8.0. The maximal amount of the product was 1.1 g of gluconic acid after 6 hours of conversion at pH 8.0 using 0.08 mg of the enzyme (152 U) and

9 $\mu$mol of thionine. The amount of the converted substrate was always more than 90 %.

Table 3. Parameters of glucose conversion reactors

| pH | $n_{thionine}$, $\mu$mol | $nCaCl_2$, mmol | $n_s$, mmol | T, h | $n_{H2O2}$, mmol | $n_{KOH}$, mmol | $n_E$, nmol | $TTN_E$x10$^{-6}$ | Conversion, % |
|---|---|---|---|---|---|---|---|---|---|
| 8.0 | 9.0 | 2.0 | 5.7 | 6.1 | 5.7 | 4.6 | 0.80 | 7.1 | 99 |
| 8.0 | 9.0 | 0.10 | 5.9 | 6.2 | 5.7 | 4.6 | 0.80 | 7.1 | 97 |
| 7.0 | 9.0 | 0.10 | 2.8 | 6.2 | 2.6 | 1.9 | 0.40 | 6.5 | 95 |
| 8.0 | 9.0 | 0.10 | 4.2 | 6.3 | 4.0 | 3.3 | 0.40 | 10.0 | 96 |
| 7.0 | 9.0 | 2.0 | 3.8 | 6.2 | 3.6 | 2.8 | 0.40 | 9.0 | 96 |
| 8.0 | 9.0 | 2.0 | 4.2 | 6.2 | 3.9 | 3.4 | 0.40 | 9.7 | 93 |
| 8.0 | 4.5 | 2.0 | 3.9 | 6.2 | 3.8 | 3.3 | 0.80 | 4.7 | 97 |
| 8.0 | 4.5 | 2.0 | 4.0 | 6.1 | 4.0 | 3.4 | 0.80 | 4.9 | 99 |
| 7.0 | 4.5 | 2.0 | 3.4 | 6.2 | 3.3 | 2.7 | 0.80 | 4.2 | 98 |
| 8.0 | 4.5 | 0.10 | 2.8 | 6.0 | 2.7 | 2.2 | 0.80 | 3.3 | 96 |
| 7.0 | 4.5 | 0.10 | 2.1 | 6.3 | 2.0 | 1.6 | 0.80 | 2.5 | 95 |

## Example 2

### Glucose conversion reactors with oxygen as terminal electron acceptor

The conversion of glucose performed in the model reactor without adding hydrogen peroxide. The specific conditions for each run of the reactor are listed in Table 4. Thionine (9.0 $\mu$mol) PQQGDH (162 U) and H2O2 decomposing enzyme - the horseradish peroxidase (82 U) or catalase (960 U) - were added to the reaction mixture at start. The reaction time was about 6 hours for every case. The amount of product formed during the reaction time assumed equal to the amount of added potassium hydroxide. This amount was used to calculate the turnover number of the PQQGDH and evaluate the efficiency of the process (Table 4). The higher yield of the product and higher turnover numbers of the enzyme were achieved in the case when HRP was used as hydrogen peroxide decomposing enzyme, 100 mM of CaCl2 were added at start and the reactor mixture pH was maintained at pH 8.0. In this case amount of the product was 0.13 g of gluconic acid after 6 hours of conversion using 0.085 mg of the enzyme (162 U) and 9 $\mu$mol of thionine. The conversion degree in this type of reactors was lower than in reactors with hydrogen peroxide dosage.

Table 4. Parameters of glucose conversion reactors without adding of hydrogen peroxide

| H2O2 decomposin g enzyme | pH | $nCaCl_2$, mmol | ns, mmol | T, h | $n_{KOH}$, mmol | $n_E$, nmol | $TTN_E$x10$^{-6}$ | Conversion,% |
|---|---|---|---|---|---|---|---|---|
| HRP | 7.0 | 0.10 | 0.64 | 5.4 | 0.33 | 0.85 | 0.39 | 52 |
| HRP | 8.0 | 0.10 | 0.74 | 5.6 | 0.56 | 0.85 | 0.66 | 76 |
| ANC | 7.0 | 0.10 | 0.49 | 6.1 | 0.36 | 0.85 | 0.42 | 72 |
| ANC | 8.0 | 0.10 | 0.61 | 6.0 | 0.46 | 0.85 | 0.54 | 76 |
| HRP | 7.0 | 2.0 | 0.61 | 5.9 | 0.42 | 0.85 | 0.49 | 70 |
| HRP | 8.0 | 2.0 | 0.83 | 5.9 | 0.66 | 0.85 | 0.77 | 79 |

## Example 3

### *Conversion of galactose and disaccharides*

The conversion of lactose, cellobiose or galactose performed in the model fed-batch reactor. The specific conditions for each run of the reactor are listed in Table 4. The amount of the horseradish peroxidase was 1.6 mg (165 U) for each run. The amount of product formed during the reaction time assumed equal to the amount of added hydrogen peroxide. This amount was used to calculate the turnover number of the PQQGDH and evaluate the efficiency of the process (Table 5). The degree of conversion was very high and depended on the amount of the enzyme used, the more enzyme was used the higher degree of conversion was achieved. According the calculated TTN the higher yield of conversion was achieved for more reactive substrates lactose and cellobiose, than for galactose.

Table 5. Parameters of galactose, cellobiose and lactose conversion reactors.

| Substrate | pH | $nCaCl_2$ mmol | $n_{thionine}$, $\mu$mol | ns, mmol | T, h | $n_{H2O2}$ mmol | $n_{KOH}$ mmol | $n_E$, nmol | $TTN_E$x $10^{-6}$ | Conversion % |
|---|---|---|---|---|---|---|---|---|---|---|
| galactose | 7.0 | 2.0 | 9.0 | 6.7 | 5.7 | 5.7 | 2.6 | 2.7 | 2.1 | 85 |
| cellobiose | 7.0 | 0.10 | 4.5 | 0.86 | 6.6 | 0.69 | 0.62 | 0.55 | 1.3 | 81 |
| cellobiose | 7.0 | 2.0 | 9.0 | 1.4 | 6.0 | 0.93 | 0.83 | 0.27 | 3.4 | 68 |
| lactose | 8.0 | 0.10 | 4.5 | 1.5 | 6.9 | 1.2 | 1.3 | 0.55 | 2.2 | 83 |
| lactose | 7.0 | 0.10 | 4.5 | 1.1 | 6.8 | 0.94 | 1.0 | 0.73 | 1.3 | 82 |
| lactose | 7.0 | 2.0 | 9.0 | 1.4 | 6.0 | 0.95 | 0.78 | 0.27 | 3.5 | 69 |

Example 4

Conversion of pentoses

The conversion of various pentoses performed in the model batch reactor. In this type of reactor, the substrate concentration in mixture can be rather high, so at start large dose of substrate (concentration in the range from 80mM to 220 mM) was added to the mixture. During the time of reaction if the rate of conversion was decreased more than three times of initial rate, then the additional amount of substrate was dosed to the mixture. In all the investigated cases the calculated concentration of substrate in reactor mixture do not exceed 220 mM. The specific conditions for each run of the reactor are listed in Table 6. The amount of the horseradish peroxidase was 1.6 mg (165 U) for each run. The amount of product formed during the reaction time assumed equal to the amount of added hydrogen peroxide. This amount was used to calculate the percent of the conversion, the turnover number of the PQQGDH and evaluate the efficiency of the process (Table 6). The degree of conversion depended on the amount of the enzyme used, the more enzyme was used the higher degree of conversion was achieved. According the calculated TTN the higher yield of conversion was achieved for more reactive substrate xylose.

Table 6. Parameters of pentoses conversion reactors.

| Substrate | $nCaCl_2$, mmol | $n_{thionine}$, $\mu$mol | ns, mmol | T, h | $n_{H2O2}$, mmol | $n_{KOH}$, mmol | $n_E$, nmol | $TTN_E$x$10^{-6}$ | Conversion, % |
|---|---|---|---|---|---|---|---|---|---|
| deoxyribose | 0.10 | 4.5 | 2.2 | 6.6 | 0.97 | 0.044 | 15 | 0.065 | 45 |
| deoxyribose | 2.0 | 9.0 | 3.9 | 6.1 | 1.1 | 0.063 | 7.2 | 0.15 | 28 |
| xylose | 0.10 | 4.5 | 27 | 45.3 | 22 | 19 | 10 | 2.2 | 81 |
| xylose | 2.0 | 9.0 | 11 | 6.0 | 10 | 7.0 | 5.5 | 1.8 | 92 |
| ribose | 2.0 | 4.5 | 6.6 | 4.3 | 3.2 | 0.54 | 4.3 | 0.73 | 48 |
| ribose | 0.10 | 4.5 | 6.8 | 7.2 | 3.6 | 0.65 | 4.4 | 0.82 | 53 |
| ribose | 2.0 | 9.0 | 11 | 6.3 | 5.5 | 3.2 | 5.5 | 1.0 | 52 |
| lyxose | 2.0 | 9.0 | 5.1 | 6.2 | 0.97 | 0.64 | 5.5 | 0.18 | 19 |
| lyxose | 0.10 | 4.5 | 3.7 | 10.3 | 2.3 | 1.6 | 15 | 0.15 | 62 |

Example 5

Conversion of mixture of galactooligosaccharides

Mixture of galactooligosaccharides as a powder was obtained from "Pienas LT" (Lithuania). For conversion of the mixture at pH 7.0 batch type of the reactor was used, but the mixture was added as powder in the amount of 0.5 g 3 times. The overall amount of the saccharides ($n_s$, mmol) in the reactor calculated assuming the known composition of the dry mixture and the molecular weight of the saccharides. The specific conditions for each run of the reactor are listed in Table 7. The amount of the horseradish peroxidase was 1.6 mg (165 U) for each run. The amount of product formed during the reaction time assumed equal to the amount of added hydrogen peroxide. This amount was used to calculate the percent of the conversion, the turnover number of the PQQGDH and evaluate the efficiency of the process (Table 6). The degree of conversion depended on the amount of the enzyme used, the more enzyme was used the higher degree of conversion was achieved. The HPLC analysis of the reactor mixtures showed that all the saccharides of the mixture are oxidized, but the present of oxidized substrate is higher for mono- and disaccharides, and only very small part of long chain saccharides are oxidized.

Table 7. Parameters of galactooligosaccharides mix conversion reactors.

| nCaCl$_2$, $\mu$mol | n$_{thionine}$, $\mu$mol | ns, mmol | T, h | n$_{H2O2}$, mmol | n$_{KOH}$, mmol | n$_E$, nmol | TTN$_E$x10$^{-6}$ | Conversion, % |
|---|---|---|---|---|---|---|---|---|
| 100 | 4.5 | 3.0 | 6.0 | 1.3 | 1.3 | 3.0 | 0.43 | 44 |
| 4000 | 4.5 | 3.0 | 6.0 | 1.6 | 1.9 | 3.0 | 0.54 | 55 |
| 2000 | 9.0 | 4.7 | 6.1 | 1.7 | 1.5 | 2.7 | 0.61 | 35 |

The experiments described in the examples above showed the ability of the enzyme dehydrogenase to oxidize the saccharides and their mixture at the maximum velocity despite the inhibition of the enzyme by the substrate. The product of the reaction - corresponding aldonic acid was neutralized by the pH sensor-controlled alkali supplement. The oxidation of the reduced enzyme was performed with the mediator thionine. The mediator was selected due to its high reactivity with dehydrogenase and peroxidase, the stability of its oxidized and reduced forms. In turn the mediator was oxidized by dioxygen or with peroxidase and hydrogen peroxide. The sensor-controlled supplement of hydrogen peroxide was developed to avoid the inactivation of the enzymes with this compound.

**References**

1. Anastassiadis S, Morgunov IG. Gluconicacid production. Recent Pat Biotechnol. 2007; 1(2):167-80. doi: 10.2174/187220807780809472. PMID: 19075839

2. Tetianec L, Bratkovskaja I, Kulys J, Časaitė V, Meškys R.. Probing Reactivity of PQQ-Dependent Carbohydrate Dehydrogenases Using Artificial Electron Acceptor. Appl biochem biotechnol. 2010; 163:404-14. doi: 10.1007/s12010-010-9048-3

3. Olsthoorn AJ, Duine JA. Production, characterization, and reconstitution of recombinant quinoprotein glucose dehydrogenase (soluble type; EC 1.1.99.17) apoenzyme of Acinetobacter calcoaceticus. Arch Biochem Biophys. 1996 ;336(1):42-8. doi: 10.1006/abbi.1996.0530. PMID: 8951033.

4. US2007/0027341

5. US5132452

6. US4843173

7. US7982031

8. US3862005

9. JP2001245657

10. JP5100987

11. WO2010/106230

12. US9315834

13. JP2007535331

14. JP2005504554

15. JP6321857

16. US 2020/0239511
17. JP2005504554
18. JP6321857
19. EP1753297
20. US4460686

**Claims**

1. A method of enzymatic conversion of saccharides to corresponding aldonic acids, said method comprising the steps of:

    a) choosing the batch or fed-batch type of the reactor and concentration of saccharides in reactor;
    b) oxidation of saccharides with the oxidized form of PQQ-dependent dehydrogenase;
    c) regeneration of oxidized form of PQQ-dependent dehydrogenase with the mediator;
    d) the reoxidation of the mediator;
    e) optionally, continuing to supplement hydrogen peroxide;
    f) optionally, continuing to supplement substrate;
    g) continuous supplementation of alkali to maintain the pH.

2. The method according to claim 1, wherein the PQQ-dependent dehydrogenase is glucose dehydrogenase.

3. The method according to claim 1, wherein the mediator is thionine.

4. The method according to claim 1, wherein the mediator reoxidation process comprises using peroxidase or catalase.

5. The method according to claim 1, wherein the mediator enzymatic reoxidation process comprises using peroxidase enzyme and continuous supplementation of hydrogen peroxide.

6. The method according to claim 1, wherein the saccharide is selected from glucose, galactose, lactose, cellobiose, pentoses, or mixture of galactooligosaccharides.

7. The method according to claims 1 to 5, wherein the substrate is supplied at the beginning of the process for batch type reactors.

8. The method according to claims 1 to 5, wherein the continuous supplementation of the substrate is used for fed-batch type reactors.

Fig. 1.

Fig. 2.

Fig. 3.

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KULYS, J. ET AL.: "Pyrroloquinoline quinone-dependent carbohydrate dehydrogenase: Activity enhancement and the role of artificial electron acceptors", BIOTECHNOLOGY JOURNAL – BIOCATALYSIS, 12 August 2010 (2010-08-12), pages 822-828, XP055963578, Retrieved from the Internet: URL:https://onlinelibrary.wiley.com/doi/epdf/10.1002/biot.201000119 [retrieved on 2022-09-21] * the whole document * * see especially: * * page 825, column 2, line 5 – page 826, column 1, line 11 * * equations 4-7 * | 1-8 | INV. C12P7/58 C12N9/04 |
| A | YOO, E.H. & LEE, S.-Y.: "Glucose Biosensors: An Overview of Use in Clinical Practice", SENSORS, vol. 10, no. 5, 4 May 2010 (2010-05-04), pages 4558-4576, XP055424345, DOI: 10.3390/s100504558 * page 4562, paragraph 3.2 * | 1-8 | |

**TECHNICAL FIELDS SEARCHED (IPC)**

C12P
C12N

The present search report has been drawn up for all claims

| Place of search | Date of completion of the search | Examiner |
|---|---|---|
| Berlin | 22 September 2022 | Fuchs, Ulrike |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 .................................................................................

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070027341 A
- US 5132452 A
- US 4843173 A
- US 7982031 B
- US 3862005 A
- JP 2001245657 B
- JP 5100987 B
- WO 2010106230 A
- US 9315834 B
- JP 2007535331 B

- JP 2005504554 B
- JP 6321857 B
- US 20200239511 A
- EP 1753297 A
- US 4460686 A
- US 5132452 B
- US 4843173 B
- US 3862005 B
- JP 2007535331 A

### Non-patent literature cited in the description

- Gluconic Acid Production. *Recent Patents on Biotechnology,* 2007, vol. 1 (2
- **OLSTHOORN ; DUINE.** *Arch. Biochem. Biophys.,* 1996, vol. 336 (1), 42
- **A. J. OLSTHOORN ; J. A. DUINE.** *Arch. Biochem. Biophys.,* 1996, vol. 336 (1), 42
- **TETIANEC et al.** *Appl. Biochem. Biotechnol.,* 2011, vol. 163, 404
- **ANASTASSIADIS S ; MORGUNOV IG.** Gluconicacid production. *Recent Pat Biotechnol,* 2007, vol. 1 (2), 167-80

- **TETIANEC L, BRATKOVSKAJA I, KULYS J, CASAITE V, MESKYS R.** Probing Reactivity of PQQ-Dependent Carbohydrate Dehydrogenases Using Artificial Electron Acceptor. *Appl biochem biotechnol.,* 2010, vol. 163, 404-14
- **OLSTHOORN AJ ; DUINE JA.** Production, characterization, and reconstitution of recombinant quinoprotein glucose dehydrogenase (soluble type; EC 1.1.99.17) apoenzyme of Acinetobacter calcoaceticus. *Arch Biochem Biophys.,* 1996, vol. 336 (1), 42-8